Europäisches Patentamt

(19) European Patent Office

Office européen des brevets

(11) Numéro de publication: **0 277 071**
**A2**

(12) **DEMANDE DE BREVET EUROPEEN**

(21) Numéro de dépôt: 88400182.7

(22) Date de dépôt: 27.01.88

(51) Int. Cl.⁴: **C 12 P 21/00**
**G 01 N 33/577, A 61 K 39/42**

(30) Priorité: 30.01.87 LU 86752

(43) Date de publication de la demande:
03.08.88 Bulletin 88/31

(84) Etats contractants désignés:
AT BE CH DE ES FR GB GR IT LI NL SE

(71) Demandeur: **IRE-CELLTARG S.A.**
**Zone Industrielle**
**B-6220 Fleurus (BE)**

(72) Inventeur: **Bron, Dominique, Dr.**
**28a, Avenue de la Sapinière**
**B-1150 Bruxelles (BE)**

**Strijckmans, Pierre**
**52, rue Plas**
**B-1860 Meise (BE)**

**Delforge, Alain**
**64, avenue des Grands Prix**
**B-1150 Bruxelles (BE)**

(74) Mandataire: **Warcoin, Jacques et al**
**Cabinet Régimbeau 26, avenue Kléber**
**F-75116 Paris (FR)**

(54) **Procédé de production d'anticorps monoclonaux humains (IgG) anti-cytomégalovirus et anticorps monoclonaux ainsi obtenus.**

㉗ La présente invention vise à fournir un procédé de production amélioré qui permet notamment de se débarrasser du génome EBV et qui fournit donc un anticorps monoclonal utile non seulement dans des tests de sérodiagnostic du cytomégalovirus (CMV) mais aussi potentiellement utile in vivo dans la prophylaxie et/ou le traitement des infections à CMV. L'invention s'étend également à la détection du virus dans les divers tissus de biopsies ou lavage bronchoalvéolaire par cet anticorps monoclonal.

EP 0 277 071 A2

## Description

Procédé de production d'anticorps monoclonaux humains (IgG) anti-cytomégalovirus et anticorps monoclonaux
ainsi obtenus

### Arrière-plan technologique

Le cytomégalovirus (CMV) est responsable d'infections atteignant surtout les nouveaux-nés (le plus souvent transmises de la mère à l'enfant), et les sujets adultes immunodéprimés.

Le cytomégalovirus est responsable, chez l'homme : 1° de syndromes mononucléosiques, qui surviennent volontiers chez les sujets ayant reçu des transfusions massives ; 2° d'atteinte hépatique, avec un tableau clinique superposable à celui de l'habituelle hépatite virale ; 3° d'infections généralisées, survenant chez des sujets présentant une tumeur maligne ou soumis à des thérapeutiques immunosuppressives ; 4° de la maladie du nouveauné, classique maladie des inclusions cytomégaliques, due à l'infection du foetus in utero. L'évolution est, en règle générale, mortelle.

Toutes ces pathologies sont réunies par les mêmes éléments du diagnostic : parfois par l'isolement du virus dans les urines, la salive ou dans tout tissu prélevé par biopsie ou postmortem ; surtout par le sérodiagnostic, qui montre une ascension du taux des anticorps entre deux prélèvements effectués à 10 ou 15 jours d'intervalle.

L'utilisation d'anticorps monoclonaux humains anti-cytomégalovirus présente donc un grand intérêt pour de tels sérodiagnostics.

EMANUEL D. et al. Human monoclonal antibody to cytomegalovirus (CMV) - J. Immunol. 4 : 2202-2206, 1984 ont déjà décrit un anticorps monoclonal humain. Cet anticorps n'a pas pu trouver une exploitation clinique et pharmaceutique, essentiellement par suite du fait qu'il conserve le génome du Epstein-Barr virus (EBV) et qu'il est d'un titre peu élevé, ce qui le rend difficilement commercialisable.

### Objet de la présente invention

La présente invention vise à fournir un procédé de production amélioré qui permet notamment de se débarasser du génome EBV et qui fournit donc un anticorps monoclonal utile non seulement dans des tests de sérodiagnostic du cytomégalovirus (CMV) mais aussi potentiellement utile in vivo dans la prophylaxie et/ou le traitement des infections à CMV. L'invention s'étend également à la détection du virus dans les divers tissus de biopsies ou lavage bronchoalvéolaire par cet anticorps monoclonal.

### Eléments caractéristiques de l'invention

La technique mise en oeuvre selon la présente invention comporte essentiellement les étapes opératoires suivantes :

a) enrichissement en lymphocytes B d'une population cellulaire mononucléée prélevée dans le sang périphérique d'un donneur ayant en une infection récente à CMV ;

b) transformation de la population cellulaire enrichie, par l'Epstein-Barr virus ;

c) établissement de lignées lymphoblastoïdes et clonage de celles produisant une forte réaction positive au test de spécificité anti-CMV ;

d) mise en culture d'une lignée choisie et fusion avec un hétéromyélome, de préférence, la lignée cellulaire SHM-D33 et sélection des hybrides produisant des IgG (classe 2),λanti-CMV et clonage pour assurer la monoclonalité.

Les anticorps monoclonàux par ce procédé reconnaissent toutes les souches de CMV en possession de la Demanderesse et notamment les trois souches de référence : TOWN ; DAVIS et AD 169.

Les diverses étapes opératoires utilisées sont connues en soi, divers détails opératoires peuvent être modifiés par le praticien mettant en oeuvre ces techniques, en fonction des circonstances.

D'un point de vue pratique, la Demanderesse préconise cependant l'exécution des différentes étapes opératoires de production dans les conditions suivantes :

La séparation de l'étape a) se déroule, de préférence, en deux stades, à savoir d'abord séparation des cellules mononucléées du sang des globules rouges sur gradient de Ficoll-Hypaque et ensuite séparation dans les lymphocytes recueillis, des lymphocytes B et des lymphocytes T par rosettage avec des globules rouges de mouton traités par AET (2-amino-ethylisothio-monium bromide hydrobromide).

L'étape b) de transformation de la population cellulaire enrichie en lymphocytes B par l'EBV s'effectue avantageusement à partir du surnageant de culture de la lignée de Marmoset (B95-8) contenant le virus EBV transformant et non infectant.

Le test de spécificité anti-CMV de l'étape c) est, de préférence, un test ELISA.

La sélection des hybrides de l'étape d) quant à elle se réalise, de préférence, par un milieu contenant de l'hypoxantine-aminoptérine-thymidine (HAT) et de l'ouabaïne.

### Exemple d'une forme d'exécution du procédé de l'invention.

Donneur : patiente, âge : 25 ans ayant subi une infection récente à CMV - titre anti-CMV : 1/40.000 (ELISA-BEHRING).

### 1ère partie : technique de production d'une lignée lymphoblastoïde anti-CMV :

30 cc de sang périphérique sont prélevés lors d'une prise de sang de routine et collectés sur tube calparinés en août 1985. Les cellules mononucléées du sang sont séparées des globules rouges sur gradient de Ficoll-Hypaque et $4 \times 10^7$ lymphocytes sont recueillis. Les lymphocytes B ont ensuite été séparés des lymphocytes T par rosettage avec des globules rouges de mouton traités par AET (2-amino-éthylisothio-uronium bromide hydrobromide). La population cellulaire enrichie en lymphocytes B (5 x $10^6$) est alors transfectée par l'EBV à partir du surnageant de culture de la lignée de Marmoset (B95-8) contenant le virus EBV transformant et non infectant, ce surnageant étant ajouté à la concentration de 20% dans le milieu de culture (Iscove's + 20% FCS).

Après 2 semaines en culture, plusieurs lignées lymphoblastoïdes sont établies et testées pour la spécificité anti CMV. Quatre de ces lignées s'avèrent positives pour la production d'anticorps anti-CMV et sont clonées par dilutions successives jusqu'à 1 cellule par puits dans une boîte à 96 puits. Plus de 50 lignées lymphoblastoïdes anti-CMV sont ainsi obtenues (septembre 1985).

2ème partie : production d'hybridomes humains anti-CMV

Une de ces lignées produisant une forte réaction positive au ELISA contre le CMV est mise en culture et $4 \times 10^7$ de ces cellules sont fusionnés avec $2 \times 10^7$ cellules d'hétéromyélome (SHM-D33) en présence de polyéthylène-glycol. Après fusion, les cellules sont déposées dans une boîte de culture à 96 puits à raison de $2 \times 10^5$ cellules par puits. Les hybrides sont sélectionnés par un milieu contenant de l'hypoxantine-aminoptérine-thymidine (HAT) et $5 \times 10^{-7}$M d'ouabaïne. En octobre 1985, les hybrides apparaissent dans 37% des puits (141/384) dont 66% produisent un anticorps monoclonal spécifique anti-CMV (ELISA-BEHRING). Tous les hybrides produisent des IgG (classe 2), λ anti-CMV dont le taux de production varie de 0,1 à 20 (median 2) ug/ml/$10^6$ cell/jour. Deux hybridomes sont clonés pour assurer la monoclonalité et 1 clone (IB-8E9H5) est sélectionné pour des tests plus spécifiques. De multiples clones sont congelés et seront testés dans un deuxième temps. L'anticorps monoclonal produit est une IgG2, λ qui reconnaît toutes les souches de CMV (y compris les 3 souches de référence : TOWN - DAVIS - AD169) dont la production est stable depuis plus de 24 mois en culture continue, dont l'activité cytotoxique pour des fibroblastes infectés par le CMV a été démontrée par un test au $^{51}$Cr en présence de complément et dont le génome EBV recherché par sonde après hybridation du DNA a disparu après fusion.

Cette dernière caractéristique est particulièrement importante car elle permet l'administration in vivo sans craindre une contamination par l'ADN de l'EBV.

L'antigène viral reconnu par l'anticorps est un antigène précoce ("early antigen") nucléaire de 64000 daltons. Des anticorps peuvent être utilisés selon toutes les techniques de diagnostic immunologique connues et peuvent notamment être présentés sous forme de trousse de détection.

DEPOT D'UN CLONE REPRESENTATIF DE L'INVENTION

Le clone IB-8E9H5 a été déposé le 5 janvier 1988 à l'ECACC (European Collection of Animal Cell Cultures -Porton Down, Salisbury, Wiltshire SP4 OJG - UNITED KINGDOM) sous la référence 88010701.

## Revendications

1. Procédé de production d'anticorps monoclonaux humains (IgG) anti-cytomégalovirus caractérisé par les étapes opératoires :

a) enrichissement en lymphocytes B d'une population cellulaire mononucléées prélevée dans le sang périphérique d'un donneur ayant subi une infection récente au CMV ;

b) transformation de la population cellulaire enrichie, par l'Epstein-Barr virus ;

c) établissement de lignées lymphoblastoïdes et clonage de celles produisant une forte réaction positive au test de spécificité anti-CMV ;

d) mise en culture d'une lignée choisie et fusion avec un hétéromyélome, de préférence, la lignée cellulaire SHM-D33 et sélection des hybrides produisant des IgG (classe 2), λ anti-CMV et clonage pour assurer la monoclonalité.

2. Procédé selon la revendication 1, caractérisé en ce que la séparation de l'étape a) se déroule en deux stades, à savoir d'abord séparation des cellules mononucléées du sang des globules rouges sur gradient de Ficoll-Hypaque et ensuite séparation dans les lymphocytes recueillis, des lymphocytes B et des lymphocytes T par rosettage avec des globules rouges de mouton traités par AET (2-aminoéthylisothio-monium bromide hydrobromide).

3. Procédé selon la revendication 1, caractérisé en ce que la transformation de l'étape b) de la population cellulaire enrichie en lymphocytes B par l'EBV s'effectue à partir du surnageant de culture de la lignée de Marmoset (B95-8) contenant le virus EBV transformant et non infectuant.

4. Procédé selon la revendication 1, caractérisé en ce que le test de spécificité anti-CMV de l'étape c) est un test ELISA.

5. Procédé selon la revendication 1, caractérisé en ce que la sélection des hybrides de l'étape d) quant à elle se réalise par un milieu contenant de l'hypoxantine-aminoptérine-thymidine (HAT) et de l'ouabaïne.

6. Anticorps monoclonal obtenu par le procédé d'une quelconque des revendications précédentes, caractérisé par la nature humaine des immunoglobulines (gammaglobuline de type Ig G) d'une part et d'autre part par sa capacité de reconnaître les souche de référence de CMV : TOWN, DAVIS et AD169 et l'absence du génome EBV.

7. Trousse de détection du cytomégalovirus caractérisée en ce qu'elle contient au moins un anticorps monoclonal selon la revendication 6.

8. Produit pharmaceutique applicable in vivo à la prophylaxie et/ou au traitement des infections à cytomégalovirus contenant un anticorps selon la revendication 6.